# EUROPEAN PATENT APPLICATION

(11) **EP 4 803 084 A1**
(43) Date of publication of application: **09.09.2026**
(21) Application number: 25305296.3
(22) Date of filing: 06.03.2025
(51) Int. Cl.: A61K 31/663, A61K 31/675, A61P 13/10, A61P 29/00, C07F 9/659

(54) **COMPOUNDS FOR USE IN THE TREATMENT OF DISEASES INVOLVING BLADDER INFLAMMATION**

(71) Applicant: IMD-Pharma, 31077 Toulouse (FR)
(72) Inventor: TURRIN, Cédric-Olivier, 31400 TOULOUSE (FR); CLEMENT, Emily, 31770 COLOMIERS (FR); OUKHRIB, Abdelouahd, 40000 MARRAKECH (MA)
(74) Representative: Icosa

(57) **Abstract**

The invention relates to aza-bisphosphonate dendrimers, or their pharmaceutically acceptable salts thereof, for use in the treatment and/or prevention of a disease or disorder involving bladder inflammation.

## Description

The present invention relates to dendrimer compounds for use in the treatment and/or prevention of a disease or disorder involving bladder inflammation, in particular interstitial cystitis and/or bladder pain syndrome.

### BACKGROUND OF THE INVENTION

Interstitial cystitis (IC), also known as bladder pain syndrome (BPS), is a chronic condition characterized by visceral pain and voiding symptoms. IC/BPS affects millions of individuals worldwide, predominantly women, and significantly impacts quality of life, leading to physical, emotional, and social challenges. IC/BPS is still an unsolved enigma with ineffective diagnosis criteria and treatment. Current treatment options, which include dietary modifications, physical therapy, and pharmacological interventions, often yield limited success and are associated with adverse effects. None of these treatments is capable of permanently resolving the inflammation and rebalancing the immune system responsible for the chronicity of the disease.

There is therefore a need for new compounds that are of therapeutic value for the treatment and/or prevention of diseases involving bladder inflammation, in particular interstitial cystitis and/or bladder pain syndrome.

Dendrimers are macromolecules consisting of monomers combined together to form a three-dimensional multi-branched architecture with a defined structure. Dendrimers have a perfectly defined size and structure thanks to their iterative synthesis. Their multivalence makes them very attractive nano-objects for many applications, especially in biology and medicine.

### SUMMARY OF THE INVENTION

The inventors have now demonstrated that aza-bisphosphonate dendrimers have the ability to reduce the pain, preserve the urothelium and reduce inflammation in bladder pain syndrome. Decreased inflammation is evidenced by (1) a reduction in edema (submucosal and muscular) visible on histological sections in treated animals, (2) reduction in cellular infiltrates in histological sections in treated animals, and (3) modulation, in bladder tissues, of the expression of RNA encoding inflammatory messengers in treated animals.

The invention therefore relates to aza-bisphosphonate dendrimers, or their pharmaceutically acceptable salts thereof, for use in the treatment and/or prevention of a disease or disorder involving bladder inflammation.

In a general aspect, the invention provides a compound of general Formula I: 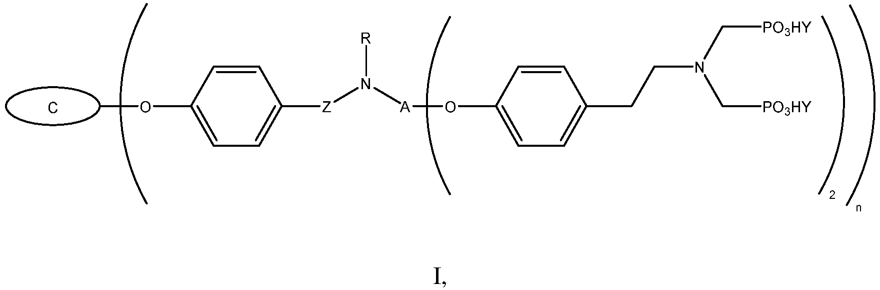 or a pharmaceutically acceptable salt thereof,
wherein 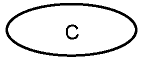 is selected from the group consisting of 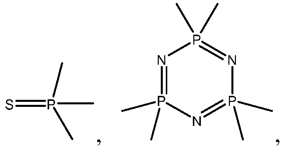 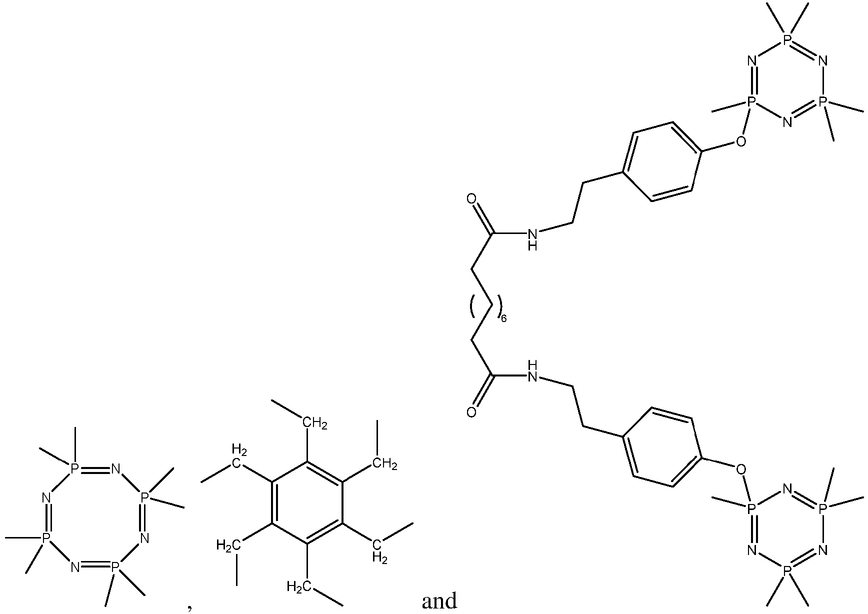
**Z** is -CH₂- or -CH=N-;
**R** is H or C1-C12-alkyl;
A is 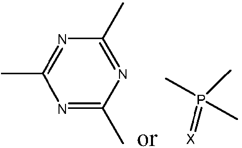 wherein **X** is S or O;
**Y** is selected from the group consisting of H, Na and K; and
**n** is an integer from 3 to 10;
for use in the treatment and/or prevention of a disease or disorder involving bladder inflammation.

The present invention also relates to a pharmaceutical composition comprising a compound of formula I as defined above, or a pharmaceutically acceptable salt thereof, and at least one pharmaceutically acceptable carrier, diluent, excipient and/or adjuvant, for use in the treatment and/or prevention of a disease or disorder involving bladder inflammation.

In another aspect, the present invention also relates to a compound of Formula VI: 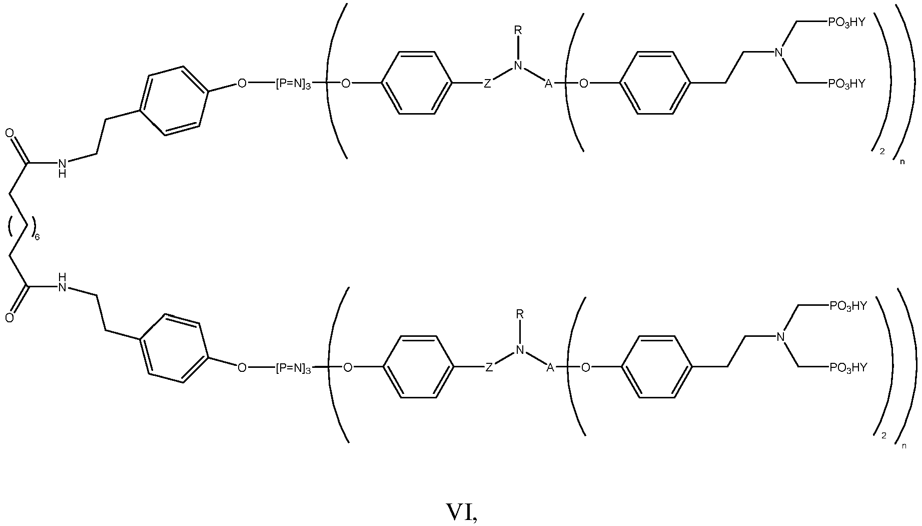 or a pharmaceutically acceptable salt thereof,
wherein
**Z** is -CH₂- or -CH=N-;
**R** is H or C1-C12-alkyl;
A is 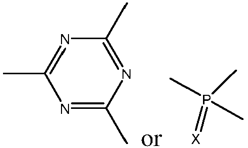 wherein **X** is S or O;
**Y** is selected from the group consisting of H, Na and K; and
n is an integer from 3 to 10.

### DETAILED DESCRIPTION OF THE INVENTION

As detailed above, the invention relates to compounds of Formula I, as well as their pharmaceutically acceptable salts, for use in the treatment and/or prevention of a disease or disorder involving bladder inflammation.

Particular compounds for use of Formula I, or pharmaceutically acceptable salts thereof, are those wherein one or more of 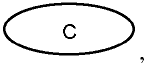 **Z, R,** A, Y and n are defined as follows: 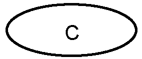 is selected from the group consisting of 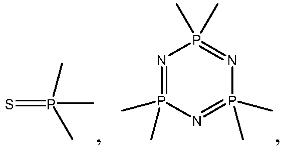 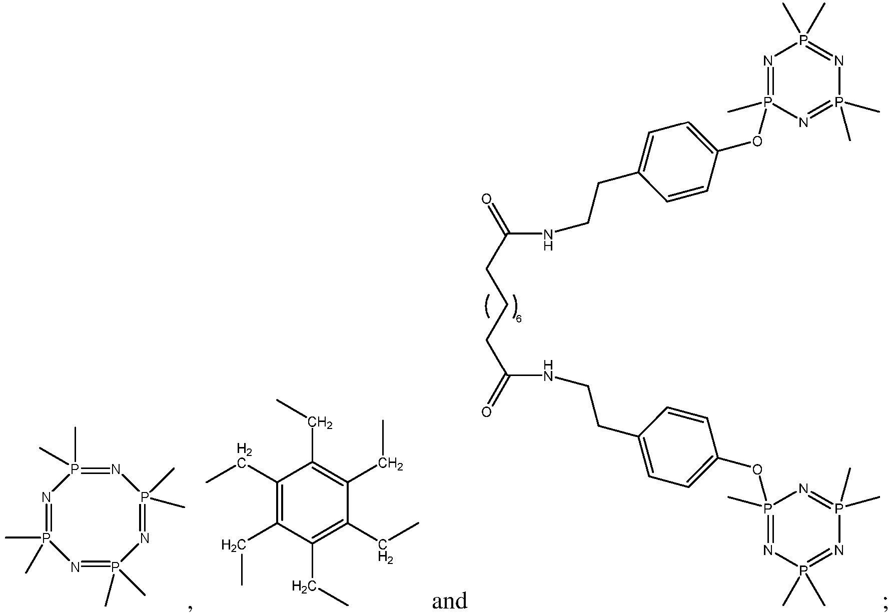 in particular 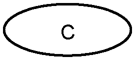 is selected from the group consisting of 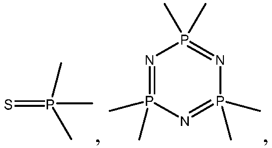 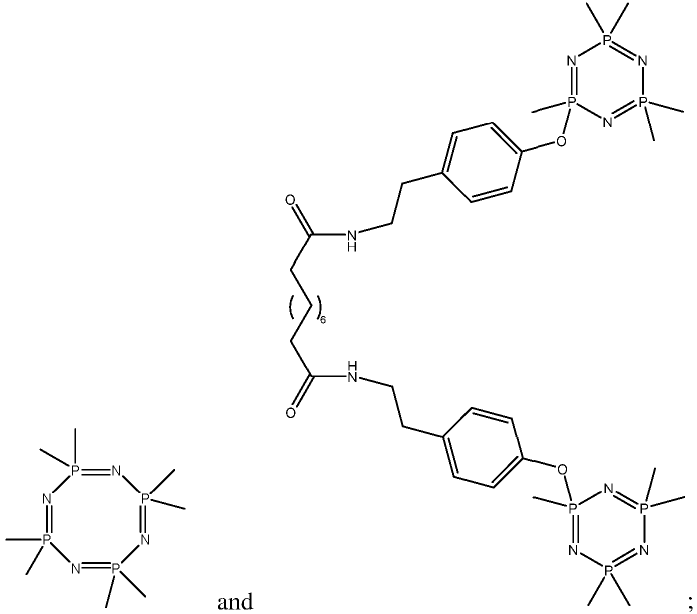 more particularly 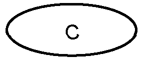 is selected from the group consisting of 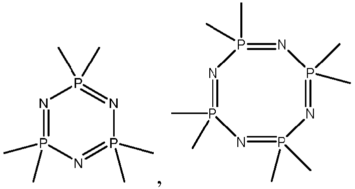 and 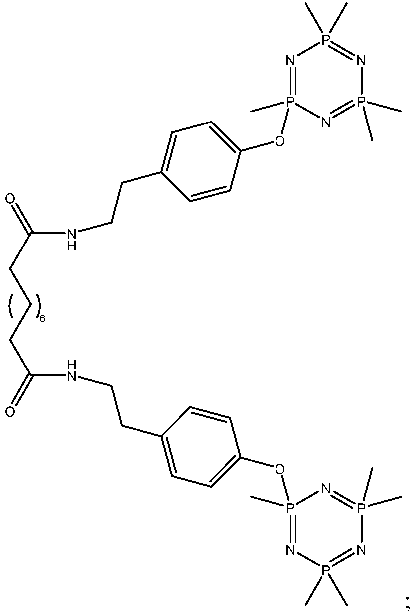 still more particularly 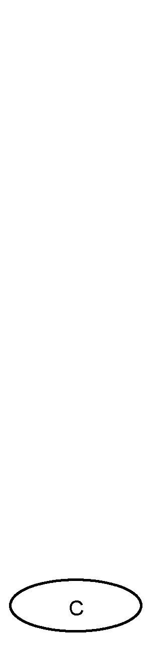 is 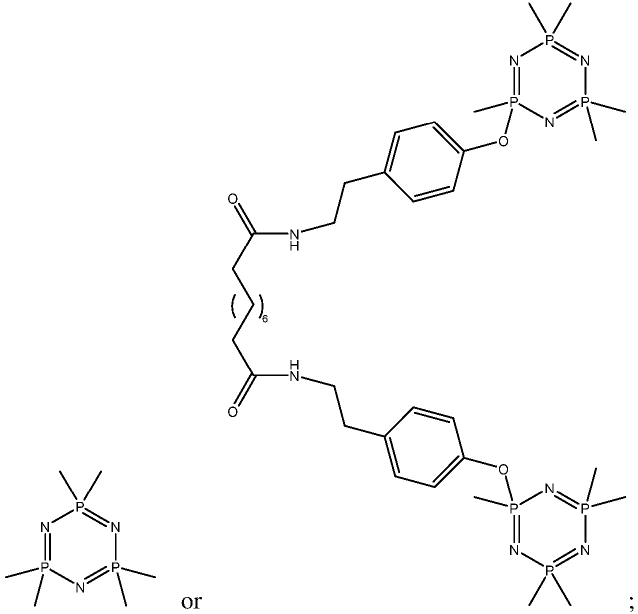 even more particularly 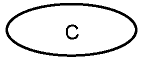 is 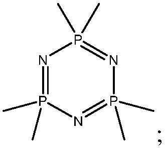
**Z** is -CH₂- or -CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
**A is** 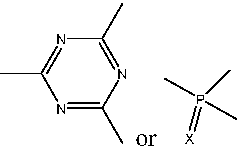 wherein **X** is S or O; in particular A is 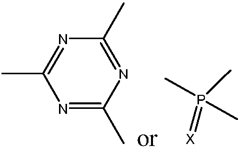
wherein **X** is S; more particularly **A** is 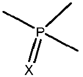 wherein **X** is S or O; even more particularly
**A is** 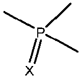 wherein X is S;
**Y** is selected from the group consisting of H, Na and K; in particular **Y** is Na or K; more particularly **Y** is Na; and
**n** is an integer from 3 to 10; in particular **n** is an integer from 4 to 10; more particularly **n** is an integer from 5 to 10.

In one embodiment, the compounds for use of Formula I are those wherein 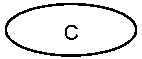 is

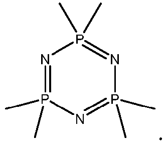

In one embodiment, the compounds for use of Formula I are those wherein 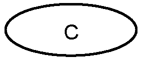 is

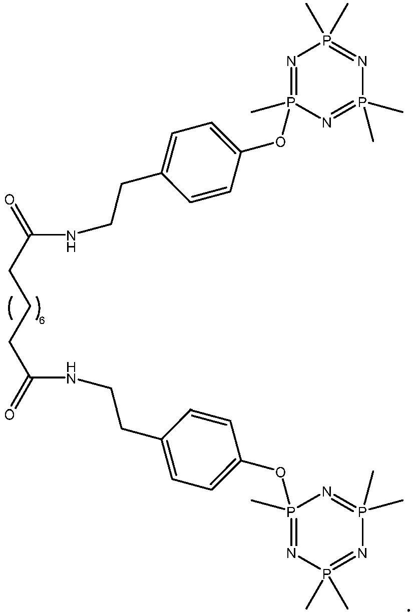

In one embodiment, the compounds for use of Formula I are those wherein **Z** is -CH₂-.

In one embodiment, the compounds for use of Formula I are those wherein **Z** is -CH=N-.

In one embodiment, the compounds for use of Formula I are those wherein **R** is methyl.

In one embodiment, the compounds for use of Formula I are those wherein **R** is n-hexyl.

In one embodiment, the compounds for use of Formula I are those wherein **R** is n-octyl.

In one embodiment, the compounds for use of Formula I are those wherein **A** is 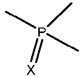 wherein X is S or O.

In one embodiment, the compounds for use of Formula I are those wherein **A** is wherein **X** is S.

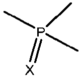

In one embodiment, the compounds for use of Formula I are those wherein **A** is 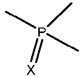 wherein **X** is O.

In one embodiment, the compounds for use of Formula I are those wherein **A** is

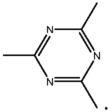

In one embodiment, the compounds for use of Formula I are those wherein **Y** is Na.

In one embodiment, the compounds for use of Formula I are those wherein **Y** is H.

In one embodiment, the compounds for use of Formula I are those wherein **n** is 6.

In one embodiment, the compounds for use of Formula I are those wherein **n** is 5.

In the present disclosure, the term "[P=N]₃" is a simplified representation of the moity

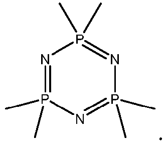

In one embodiment, the compounds for use of Formula I are those of Formula II: 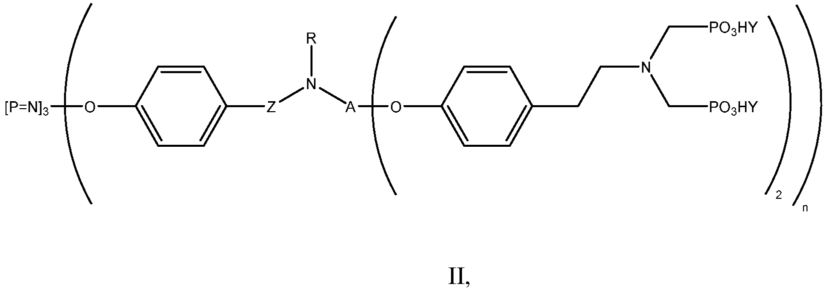 or pharmaceutically acceptable salts thereof,
wherein **Z, R, A, Y** and **n** are as defined above with respect to Formula I and any of its embodiments.

Particular compounds for use of Formula II, or pharmaceutically acceptable salts thereof, are those wherein one or more of **Z, R, A, Y** and **n** are defined as follows:
**Z** is -CH₂- or -CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
**A** is 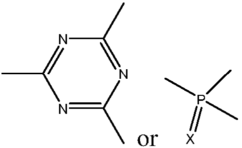 wherein **X** is S or O; in particular **A** is 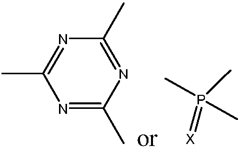
wherein **X** is S; more particularly **A** is 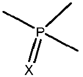 wherein **X** is S or O; even more particularly
**A** is 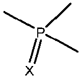 wherein **X** is S;
**Y** is selected from the group consisting of H, Na and K; in particular **Y** is Na or K; more particularly **Y** is Na; and
**n** is an integer from 3 to 10; in particular **n** is an integer from 4 to 8; more particularly **n** is an integer from 5 to 7; even more particularly **n** is 6.

In one embodiment, the compounds for use of Formula I are those of Formula III: 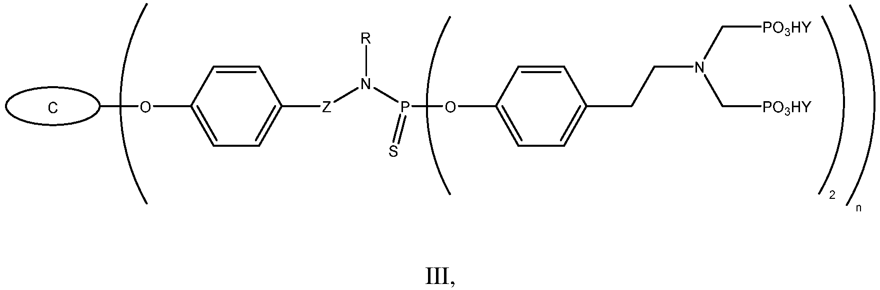 or pharmaceutically acceptable salts thereof,
wherein 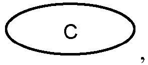 **Z, R, Y** and **n** are as defined above with respect to Formula I and any of its embodiments.

In one embodiment, the compounds for use of Formula I are those of Formula IV: 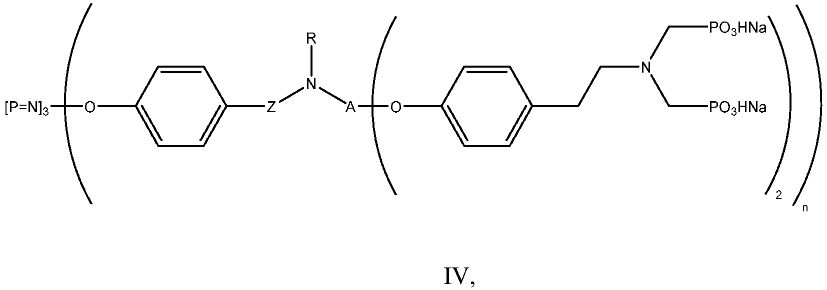 or pharmaceutically acceptable salts thereof,
wherein **Z, R, A** and **n** are as defined above with respect to Formula I and any of its embodiments.

Particular compounds for use of Formula IV, or pharmaceutically acceptable salts thereof, are those wherein one or more of **Z, R, A** and **n** are defined as follows:
**Z** is -CH₂- or -CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
**A** is 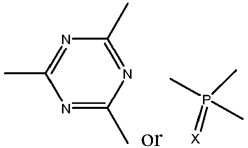 wherein **X** is S or O; in particular **A** is 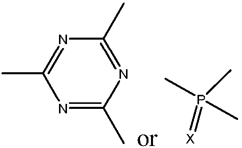
wherein **X** is S; more particularly A is 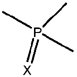 wherein **X** is S or O; even more particularly
**A** is 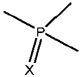 wherein **X** is S; and
**n** is an integer from 3 to 10; in particular n is an integer from 4 to 8; more particularly **n** is an integer from 5 to 7; even more particularly **n** is 6.

In one embodiment, the compounds for use of Formula I are those of Formula V: 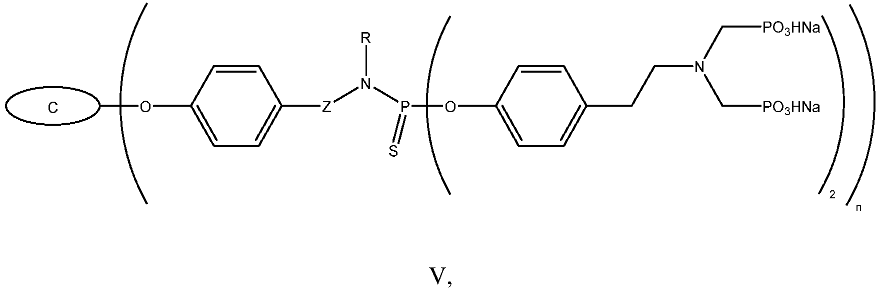 or pharmaceutically acceptable salts thereof,
wherein 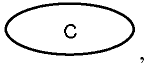 **Z, R** and **n** are as defined above with respect to Formula I and any of its embodiments.

Particular compounds for use of Formula V, or pharmaceutically acceptable salts thereof, are those wherein one or more of **Z, R, A, Y** and **n** are defined as follows:
**Z** is -CH₂- or -CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
**A** is 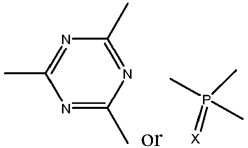 wherein **X** is S or O; in particular **A** is 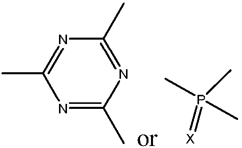
wherein **X** is S; more particularly **A** is 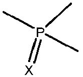 wherein **X** is S or O; even more particularly
**A** is 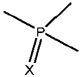 wherein **X** is S;
**Y** is selected from the group consisting of H, Na and K; in particular **Y** is Na or K; more particularly **Y** is Na; and
**n** is an integer from 3 to 10; in particular **n** is an integer from 4 to 8; more particularly n is an integer from 5 to 7; even more particularly **n** is 5.

In one embodiment, the compounds for use of Formula I are those of Formula VI: 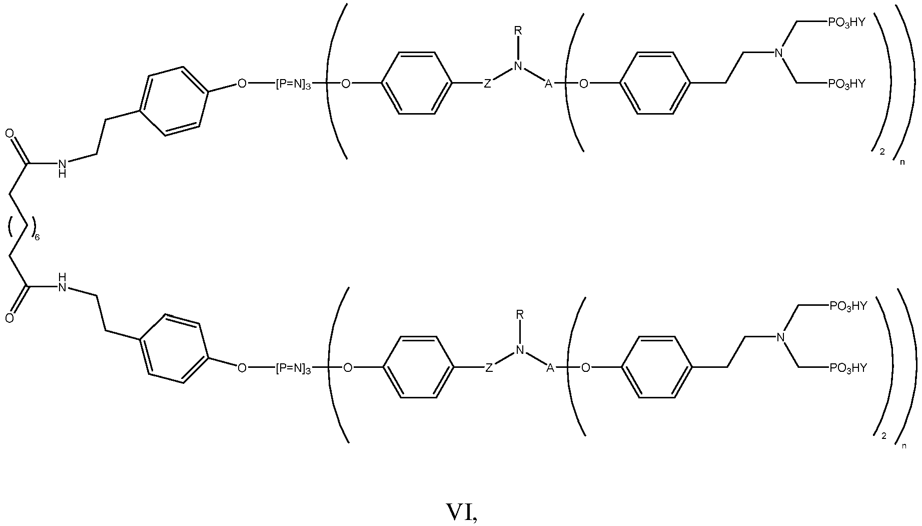 or pharmaceutically acceptable salts thereof, wherein **Z, R, A, Y** and **n** are as defined above with respect to Formula I and any of its embodiments.

Particular compounds for use of Formula VI, or pharmaceutically acceptable salts thereof, are those wherein one or more of **Z, R, A, Y** and **n** are defined as follows:
**Z** is -CH₂- or -CH=N-; in particular **Z** is-CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
A is 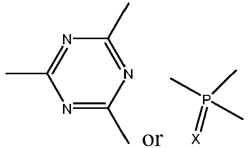 wherein **X** is S or O; in particular **A** is 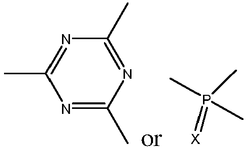
wherein X is S; more particularly A is 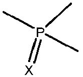 wherein **X** is S or O; even more particularly
A is 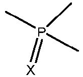 wherein **X** is S;
**Y** is selected from the group consisting of H, Na and K; in particular **Y** is Na or K; more particularly **Y** is Na; and
**n** is an integer from 3 to 10; in particular **n** is an integer from 4 to 8; more particularly **n** is an integer from 4 to 6; still more particularly **n** is 5.

Particularly preferred compounds for use of the invention are those listed in Table 1 hereafter:

**Table 1**

| **Structure** |
|---|
| 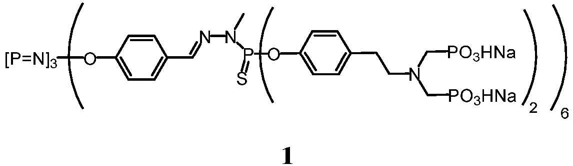 |
| 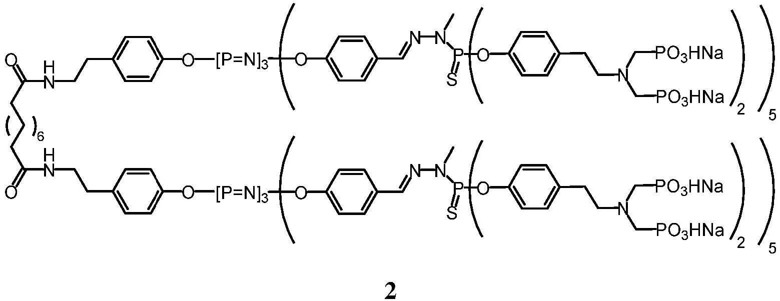 |
| 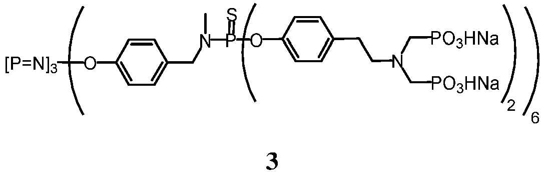 |
| 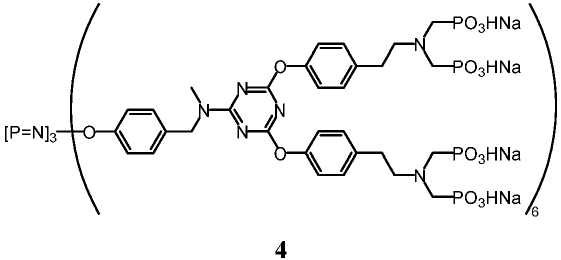 |

The compounds of the invention can be prepared in different ways with reactions known by the person skilled in the art. Reaction schemes as described in the example section illustrate by way of example different possible approaches.

The compounds of the invention have the ability to reduce the pain in a patient suffering of bladder pain syndrome.

Accordingly, in a particularly preferred embodiment, the invention relates to the use of compounds of Formula I and its sub-formulae, in particular those of Table 1 above, or pharmaceutically acceptable salts thereof, in the treatment and/or prevention of a disease or disorder involving bladder inflammation, in particular interstitial cystitis and/or bladder pain syndrome.

### APPLICATIONS

Unexpectedly, the inventors have discovered that the compounds of formula I according to the present invention have the ability to reduce pain in a chronic rat model of bladder pain syndrome. In addition, the compounds of the invention have the ability to preserve the urothelium and to reduce inflammation in a chronic rat model of bladder pain syndrome. Decreased inflammation is evidenced by (1) a reduction in edema (submucosal and muscular) visible on histological sections in treated animals, (2) reduction in cellular infiltrates in histological sections in treated animals, and (3) modulation, in bladder tissues, of the expression of RNA encoding inflammatory messengers in treated animals. As a result, the compounds of formula I according to the invention may be used to reduce the pain, to preserve the urothelium and to reduce inflammation in a patient suffering of a disease or disorder involving bladder inflammation, in particular bladder pain syndrome and/or interstitial cystitis.

The compounds of the invention are therefore useful in the prevention and/or treatment of a disease or disorder involving bladder inflammation, in particular interstitial cystitis and/or bladder pain syndrome.

The invention thus also relates to a compound of the invention, or a pharmaceutically acceptable salt thereof, for use in treating and/or preventing a disease or disorder involving bladder inflammation, in particular interstitial cystitis or bladder pain syndrome.

Diseases or disorders involving bladder inflammation within the meaning of the present invention include, but are not limited to, cystitis, urinary incontinence, overactive bladder, interstitial cystitis, bladder pain syndrome and bladder cancer, in particular cystitis, urinary incontinence, overactive bladder, interstitial cystitis and bladder pain syndrome, more particularly interstitial cystitis and bladder pain syndrome. In a particular example, the disease or disorder involving bladder inflammation is bladder pain syndrome. In another particular example, the disease or disorder involving bladder inflammation is interstitial cystitis.

Thus, in one embodiment, the invention relates to the compound of Formula I, or a pharmaceutically acceptable salt thereof, for use in treating and/or preventing a disease or disorder selected from the group consisting of cystitis, urinary incontinence, overactive bladder, interstitial cystitis, bladder pain syndrome and bladder cancer. In particular, the invention relates to a compound of the invention, or a pharmaceutically acceptable salt thereof, for use in treating and/or preventing a disease or disorder selected from the group consisting of cystitis, urinary incontinence, overactive bladder, interstitial cystitis and bladder pain syndrome. More particularly, the invention relates to a compound of the invention, or a pharmaceutically acceptable salt thereof, for use in treating and/or preventing a disease or disorder selected from the group consisting of interstitial cystitis and bladder pain syndrome. In a particular example, the invention relates to a compound of the invention, or a pharmaceutically acceptable salt thereof, for use in treating and/or preventing bladder pain syndrome. In a particular example, the invention relates to a compound of the invention, or a pharmaceutically acceptable salt thereof, for use in treating and/or preventing interstitial cystitis.

Or in other terms, the invention also relates to a method of treating and/or preventing a disease or disorder involving bladder inflammation, in particular interstitial cystitis or bladder pain syndrome, comprising the administration of a therapeutically effective amount of a compound of the invention, or pharmaceutically acceptable salt thereof, to a patient in need thereof. Preferably the patient is a warm-blooded animal, more preferably a human. The diseases or disorders involving bladder inflammation are preferably those defined above.

The invention further provides the use of a compound of the invention or a pharmaceutically acceptable salt or solvates thereof for the manufacture of a medicament for use in treating and/or preventing a disease or disorder involving bladder inflammation. Preferably the patient is a warm-blooded animal, more preferably a human. The diseases or disorders involving bladder inflammation are preferably those defined above.

In another embodiment, the invention relates to the use of a compound of the invention, or a pharmaceutically acceptable salt thereof, for reducing the pain in a patient suffering of a disease or disorder involving bladder inflammation. In other terms, the invention relates to a compound of the invention, or a pharmaceutically acceptable salt thereof, for use in reducing the pain in a patient suffering of a disease or disorder involving bladder inflammation.

In another embodiment, the invention relates to the use of a compound of the invention, or a pharmaceutically acceptable salt thereof, for preserving the urothelium in a patient suffering of a disease or disorder involving bladder inflammation. In other terms, the invention relates to a compound of the invention, or a pharmaceutically acceptable salt thereof, for use in preserving the urothelium in a patient suffering of a disease or disorder involving bladder inflammation.

In another embodiment, the invention relates to the use of a compound of the invention, or a pharmaceutically acceptable salt thereof, for reducing inflammation in a patient suffering of a disease or disorder involving bladder inflammation. In other terms, the invention relates to a compound of the invention, or a pharmaceutically acceptable salt thereof, for use in reducing inflammation in a patient suffering of a disease or disorder involving bladder inflammation.

In another embodiment, the invention relates to the use of a compound of the invention, or a pharmaceutically acceptable salt thereof, for reducing the pain, preserving the urothelium and reducing inflammation in a patient suffering of a disease or disorder involving bladder inflammation. In other terms, the invention relates to a compound of the invention, or a pharmaceutically acceptable salt thereof, for use in reducing the pain, preserving the urothelium and reducing inflammation in a patient suffering of a disease or disorder involving bladder inflammation.

The invention also provides pharmaceutical compositions comprising a compound of Formula I as defined above, or a pharmaceutically acceptable salt thereof, and at least one pharmaceutically acceptable carrier, diluent, excipient and/or adjuvant, for use in the treatment and/or prevention of a disease or disorder involving bladder inflammation.

According to one embodiment, the compounds of the invention, or their pharmaceutically acceptable salts thereof, may be administered as part of a combination therapy. Thus, are included within the scope of the present invention embodiments comprising coadministration of, and compositions and medicaments which contain, in addition to a compound of the present invention, a pharmaceutically acceptable salt thereof, as an active ingredient, additional therapeutic agents and/or active ingredients. Such multiple-drug regimens, often referred to as combination therapy, may be used in the treatment and/or prevention of any disease or disorder involving bladder inflammation.

Thus, the methods of treatment and pharmaceutical compositions of the present invention may employ the compounds of the invention, or their pharmaceutically acceptable salts thereof, in the form of monotherapy but said methods and compositions may also be used in the form of multiple therapies in which one or more compounds of Formula I, or their pharmaceutically acceptable salts thereof, are co-administered in combination with one or more other therapeutic agents.

As indicated above, the invention also covers pharmaceutical compositions which contain, in addition to a compound of the present invention, a pharmaceutically acceptable salt thereof as an active ingredient, additional therapeutic agents and/or active ingredients, for use in the treatment and/or prevention of a disease or disorder involving bladder inflammation.

Another object of this invention is a medicament comprising at least one compound of the invention, or a pharmaceutically acceptable salt thereof, as an active ingredient.

Generally, for pharmaceutical use, the compounds of the invention may be formulated as a pharmaceutical preparation comprising at least one compound of the invention and at least one pharmaceutically acceptable carrier, diluent, excipient and/or adjuvant, and optionally one or more further pharmaceutically active compounds.

By means of non-limiting examples, such a formulation may be in a form suitable for oral administration, parenteral administration (such as by intravesical, intravenous, intramuscular or subcutaneous injection or intravenous infusion), topical administration (including ocular), cerebral administration, sublingual administration, aerosol administration, for administration by inhalation, by a skin patch, by an implant, by a suppository, etc. Such suitable administration forms - which may be solid, semi-solid or liquid, depending on the manner of administration - as well as methods and carriers, diluents and excipients for use in the preparation thereof, will be clear to the skilled person; reference is made to the latest edition of Remington's Pharmaceutical Sciences.

The invention also provides a compound of Formula VI: 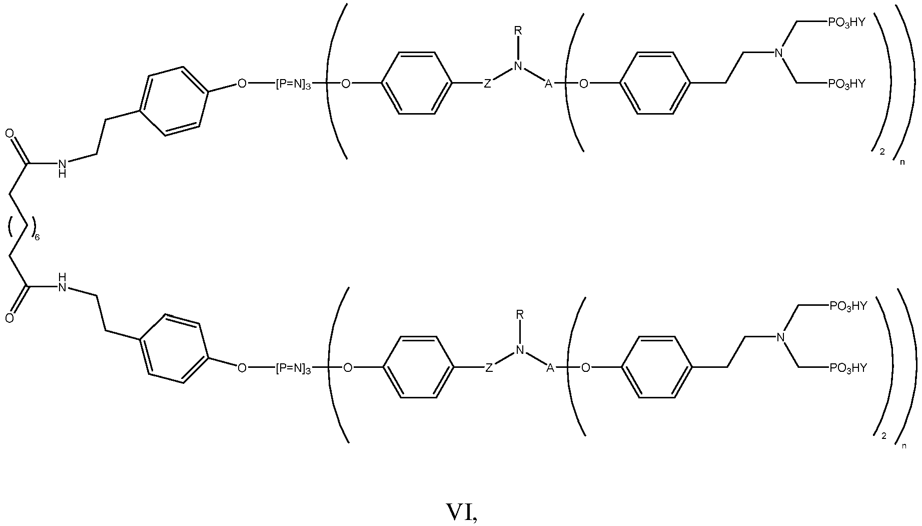 or a pharmaceutically acceptable salt thereof,
wherein
**Z** is -CH₂- or -CH=N-;
**R** is H or C1-C12-alkyl;
A is 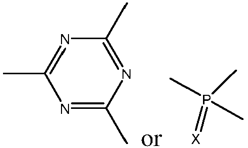 wherein **X** is S or O;
**Y** is selected from the group consisting of H, Na and K; and
**n** is an integer from 3 to 10.

Particular compounds of Formula VI, or pharmaceutically acceptable salts thereof, are those wherein one or more of **Z, R, A, Y** and **n** are defined as follows:
**Z** is -CH₂- or -CH=N-; in particular **Z** is-CH=N-;
**R** is H or C1-C12-alkyl; in particular **R** is C1-C12-alkyl, more particularly **R** is C1-C8-alkyl, even more particularly **R** is C1-C4-alkyl, still more particularly **R** is selected from methyl, n-hexyl and n-octyl; in a particular example **R** is methyl;
**A** is 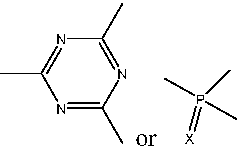 wherein **X** is S or O; in particular **A** is 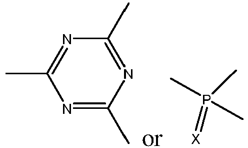
wherein **X** is S; more particularly **A** is 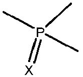 wherein **X** is S or O; even more particularly
A is 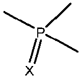 wherein X is S;
**Y** is selected from the group consisting of H, Na and K; in particular **Y** is Na or K; more particularly **Y** is Na; and
**n** is an integer from 3 to 10; in particular **n** is an integer from 4 to 8; more particularly **n** is an integer from 4 to 6; still more particularly **n** is 5.

Particularly preferred compounds of Formula VI are those listed in Table 2 hereafter:

**Table 2**

| **Structure** |
|---|
| 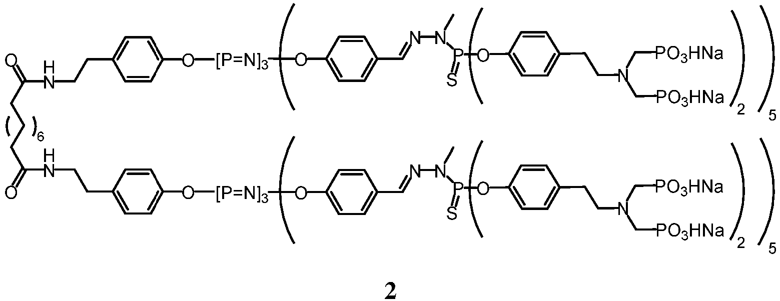 |

The compounds of the invention can be prepared in different ways with reactions known by the person skilled in the art. Reaction schemes as described in the example section illustrate by way of example different possible approaches.

### DEFINITIONS

The definitions and explanations below are for the terms as used throughout the entire application, including both the specification and the claims.

Unless otherwise stated any reference to compounds of the invention herein, means the compounds as such as well as their pharmaceutically acceptable salts and solvates.

When describing the compounds of the invention, the terms used are to be construed in accordance with the following definitions, unless indicated otherwise.

The term "halo" or "halogen" refers to the atoms of group 17 of the periodic table (halogens) and includes in particular fluorine, chlorine, bromine and iodine atoms.

The term "alkyl" by itself or as part of another substituent refers to a hydrocarbyl radical of Formula CₙH₂ₙ₊₁ wherein n is a number greater than or equal to 1.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like.

The term "cycloalkyl" as used herein is a monovalent, saturated, or unsaturated monocyclic or bicyclic hydrocarbyl group. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, and cyclohexyl.

The term "heteroatom" as used herein refers to any atom that is not carbon or hydrogen. Non-limiting examples of such heteroatoms include nitrogen, oxygen, sulfur, and phosphorus. Preferred heteroatoms are nitrogen and oxygen.

The term "aryl" as used herein refers to a polyunsaturated, aromatic hydrocarbyl group having a single ring (*i.e.* phenyl) or multiple aromatic rings fused together (e.g. naphthyl), typically containing 5 to 12 atoms; preferably 6 to 10, wherein at least one ring is aromatic. Examples of aryl groups include but are not limited to phenyl, naphthyl and anthracyl. Preferred aryl group according to the invention is phenyl.

The compounds of the invention containing a basic functional group may be in the form of pharmaceutically acceptable salts. Pharmaceutically acceptable salts of the compounds of the invention containing one or more basic functional groups include in particular the acid addition salts thereof. Suitable acid addition salts are formed from acids which form nontoxic salts. Examples include acetate, adipate, aspartate, benzoate, besylate, bicarbonate/carbonate, bisulfate/sulfate, borate, camsylate, cinnamate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Pharmaceutically acceptable salts of compounds of Formula I and sub-formulae may for example be prepared as follows:
(i) reacting the compound of Formula I or any of its sub-formulae with the desired acid; or
(ii) converting one salt of the compound of Formula I or any of its sub-formulae to another by reaction with an appropriate acid or by means of a suitable ion exchange column.

All these reactions are typically carried out in solution. The salt may precipitate from the solution and be collected by filtration or may be recovered by evaporation of the solvent. The degree of ionization in the salt may vary from completely ionized to almost non-ionized.

The term "solvate" is used herein to describe a molecular complex comprising the compound of the invention and one or more pharmaceutically acceptable solvent molecules, for example, ethanol. The term "hydrate" is employed when said solvent is water.

The compounds of the invention include compounds of the invention as hereinbefore defined, including all polymorphs and crystal habits thereof, prodrugs and isomers thereof (including optical, geometric, and tautomeric isomers) and isotopically-labelled compounds of the invention.

In addition, although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention.

The term "patient" refers to a warm-blooded animal, more preferably a human, who/which is awaiting or receiving medical care or is or will be the object of a medical procedure.

The term "human" refers to subjects of both genders and at any stage of development (*i.e.* neonate, infant, juvenile, adolescent, adult). In one embodiment, the human is an adolescent or adult, preferably an adult.

The terms "treat", "treating" and "treatment", as used herein, are meant to include alleviating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient which is sufficient to achieve the desired therapeutic or prophylactic effect in the individual to which it is administered.

The term "administration", or a variant thereof (e.g.," administering"), means providing the active agent or active ingredient, alone or as part of a pharmaceutically acceptable composition, to the patient in whom/which the condition, symptom, or disease is to be treated or prevented.

By "pharmaceutically acceptable" is meant that the ingredients of a pharmaceutical composition are compatible with each other and not deleterious to the patient thereof.

The term "agonist" as used herein means a ligand that activates an intracellular response when it binds to a receptor.

The term "pharmaceutical vehicle" as used herein means a carrier or inert medium used as solvent or diluent in which the pharmaceutically active agent is formulated and/or administered. Non-limiting examples of pharmaceutical vehicles include creams, gels, lotions, solutions, and liposomes.

The present invention will be better understood by referring to the following examples and figures. These examples are intended to be representative of specific embodiments of the invention and are not intended as limiting the scope of the invention.

### FIGURES

**Figure 1****:** Effects of CYP and effects of the compounds of the invention on AUCs (allodynia).
**Figure 2****:** Effects of CYP and effects of the compounds of the invention on AUCs (hyperalgesia).
**Figure 3****:** Effect of CYP and effects of the compounds of the invention on bladder pain (nociceptive scores).
**Figure 4****:** Effect of CYP and effects of the compounds of the invention on bladder pain (nociceptive thresholds).
**Figure 5****:** Histopathological images of bladder following H/E staining showing the effects of the compounds of the invention on edema and urothelial.
**Figure 6****:** Histopathological images of bladder following UROPLAKIN staining showing the effects of the compounds of the invention on the preservation of urothelium integrity.
**Figure 7****:** confocal imaging of urothelial SV-HUC-1 cells showing the internalization of fluorescent analogues of the compounds of the invention (dendrimer 1 and dendrimer 3) and their co-localization with mitochondria.

### EXAMPLES

### CHEMISTRY EXAMPLES

All the temperatures are expressed in °C and all the reactions were carried out at room temperature (RT) unless indicated otherwise.

The reactions were monitored using thin-layer chromatography (TLC) carried out on ready-to-use sheets of aluminum covered with a silica gel and a fluorescence indicator UV254 (Kieselgel^{®} 60 F254 Merck, 0.2 mm thick) or equivalent, or by ³¹P NMR.

Preparatory column chromatographies were carried out by the silica gel chromatography method, using silica with a particle size of 63 to 200 µm, by Sigma Aldrich.

NMR analyses were carried out on a 300 MHz, 400 MHz or 600 Mhz Bruker spectrometer. The spectra are recorded in solution in deuterated water (D₂O), deuterated chloroform (CDCl₃), in deuterated dichloromethane (CD₂Cl₂), in deuterated acetonitrile (CD₃CN), in deuterated methanol (CD₃OD) or else in deuterated dimethyl sulfoxide (DMSO-d6) or in mixtures of these deuterated solvents. The chemical shifts (δ) are given in ppm followed, for the proton spectra, by the multiplicity, where s, brs, d, t, q, dd, td, and m denote, respectively, singlets, broad singlets, doublets, triplets, quadruplets, doublets of doublets, triplets of doublets and multiplets (or incompletely resolved peaks). The multiplicities are followed, where appropriate, by the value of the coupling constants, denoted J and expressed in Hertz (Hz). All carbon (¹³C) and phosphorus (³¹P) NMR spectra were performed by removing the couplings with protons (¹³C-{¹H} NMR and ³¹P-{¹H}) NMR).

Solvents, reagents and starting materials were purchased from well-known chemicals suppliers such as Sigma Aldrich, Acros Organics, VWR International. Solvents, unless indicated otherwise, were purified by distillation before use. Reagents and starting materials, unless indicated, were used without additional purifications.

The following abbreviations are used:
AUC: Area under the curve,
BrTMS: Bromotrimethylsilane,
CYP: Cyclophosphamide,
DCM: Dichloromethane,
DMF: N,N-dimethylformamide,
DMSO: Dimethylsulfoxide,
eq: Equivalent,
ESI: Electrospray ionization,
EtOH: Ethanol,
MeOH: Methanol,
MS: Mass spectrometry,
NMR: Nuclear magnetic resonance,
rt or RT: Room temperature,
THF: tetrahydrofuran,
t_{R}: Retention time.

### Dendrimer 1

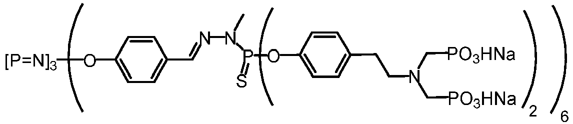

Dendrimer 1 was synthesized according to the synthetic procedure described in M. Poupot et al., FASEB J. 2006, 20, 2339-2351.

### Dendrimer 2

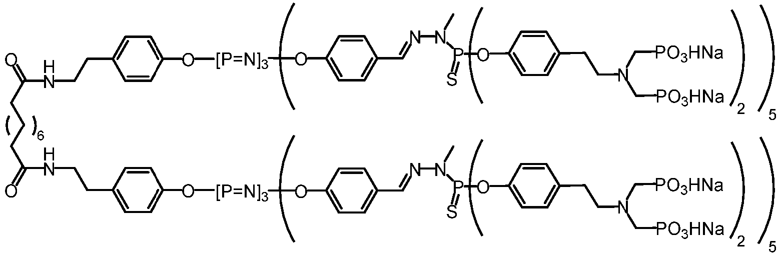

Dendrimer 2 was synthesized according to the following procedure. 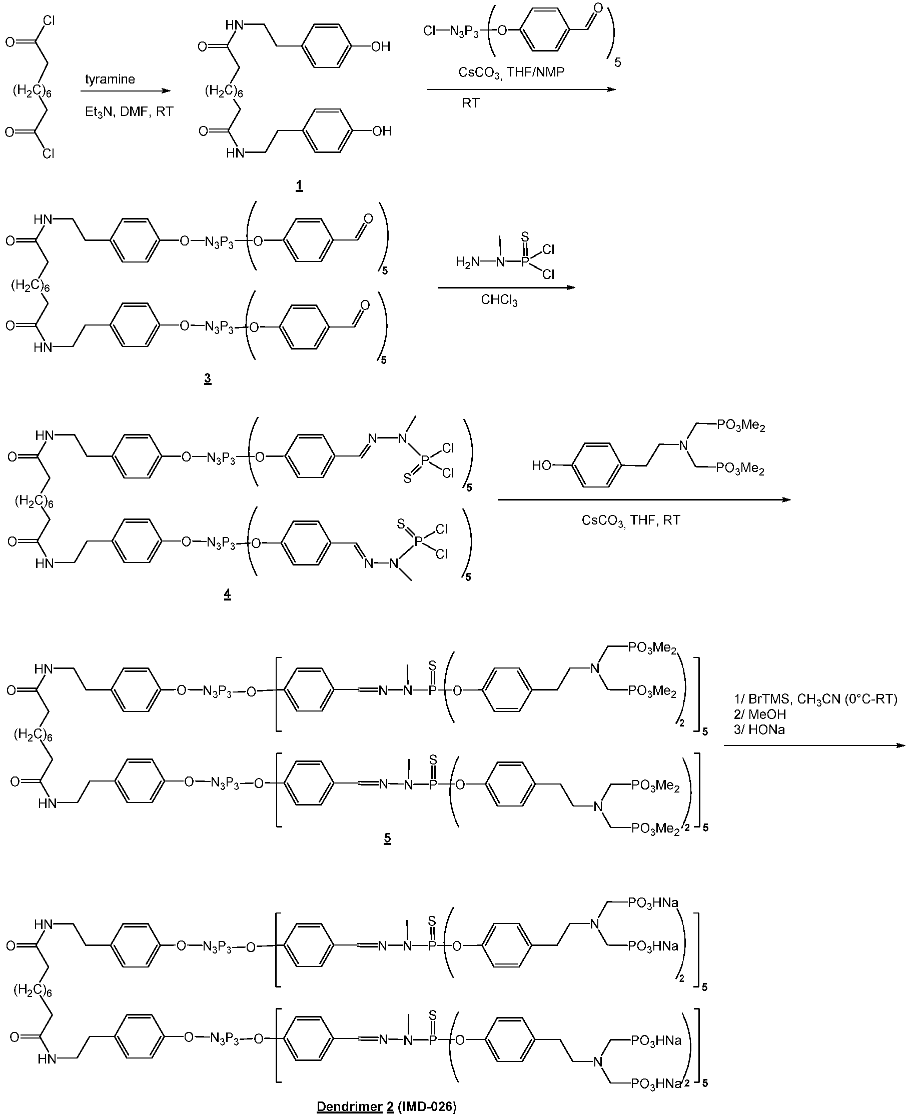 (Cl)[N₃P₃](OC₆H₄CHO)₅, HOC₆H₄CH₂CH₂N[CH₂PO₃Me₂]₂ and H₂NNMeP(S)Cl₂ were prepared according to O. Rolland et al., Chem. Eur. J. 2008, 14, 4836-4850.

To a solution of decanedioyl dichloride (5 g, 21 mmol) in DMF (50 mL) at 0°C are added HOC₆H₄CH₂CH₂N[CH₂PO₃Me₂]₂ (8.6 g, 63.02 mmol) and triethylamine (8.7 mL, 63.02 mmol). The mixture is stirred overnight at the room temperature. After removing DMF in vacuum, the crude mixture is dissolved in methanol (10 mL) and then precipitated in water (50 mL). The bisphenol **1** is obtained as with powder in 80% yield. ¹H NMR (DMSO-*d*₆) δ: 1.21 (d, J=4.0 Hz, 8H), 1.46 (t, J=7.1 Hz, 4H), 2.02 (t, J=7.4 Hz, 4H), 2.57 (t, J=8.2 Hz, 4H), 3.19 (dt, J=7.9, 5.9 Hz, 4H), 6.67 (d, J=8.4 Hz, 4H), 6.97 (d, J=8.4 Hz, 4H), 7.79 (t, J=5.6 Hz, 2H), 9.15 (s, 2H). ¹³C NMR (DMSO-d₆) δ: 25.76 (s), 29.11 (s), 29.17 (s), 34.89 (s), 35.89 (s), 40.90 (s), 115.49 (s), 129.88 (s), 130.01 (s), 156.04 (s), 172.42 (s).

To a solution of **1** (0.5 g, 1.17 mmol) dissolved in N-methylpyrolidinone (1 mL) are added CsCO₃ 1.52 g, 4.69 mmol) and (Cl)[N₃P₃](OC₆H₄CHO)₅ (2.29 g, 3.51 mmol) in THF (20 mL). The reaction mixture is stirred overnight at room temperature. Then the solution is centrifuged, filtered and then concentrated under reduced pressure. The residue is purified by column chromatography using DCM/AcOEt (40/60) to give the 3 as a transparent oil in 70% yield. ³¹P NMR (CDCl₃) δ: 7.38 (s). ¹H NMR (CDCl₃) δ: 1.28 (br s, 8H), 1.60 (t, J=7.3 Hz, 4H), 2.13 (t, J=7.6 Hz, 4H), 2.80 (t, J=7.3 Hz, 4H), 3.62-3.34 (m, 4H), 5.67 (t, J=5.9 Hz, 2H), 6.93 (d, J=8.3 Hz, 4H), 7.05 (d, J=8.5 Hz, 4H), 7.11 (d, J=8.5 Hz, 8H), 7.18 (d, J=8.5 Hz, 12H), 7.73 (t, J=8.8 Hz, 20H), 7.75 (d, J=8.6 Hz, 1H), 9.94 (s, 6H), 9.95 (s, 4H). ¹³C NMR (CDCl₃) δ: 25.58 (s), 29.03 (s), 29.12 (s), 35.09 (s), 36.66 (s), 40.49 (s), 120.70 (dd, J=3.2, J=1.6 Hz), 121.27 (d, J=2.9 Hz), 129.85 (s), 131.36 (s), 133.59 (s), 133.62 (s), 133.71 (s), 136.62 (s), 148.51 (br s), 154.61 (s), 154.80 (br s), 173.18 (s), 190.41 (s), 190.47 (s), 190.59 (s).

To a solution of 3 (1 g, 0.52 mmol) in chloroform (10 mL) is added a solution of H₂NNMeP(S)Cl₂ (0.2M) in chloroform (42 mL, 8.40 mmol). The reaction mixture is stirred for 2 hours at room temperature. Then the chloroform is evaporated in vacuum; the residue obtained is dissolved in THF (4 mL) and purified by precipitation in pentane (150 mL) to obtain the dendrimer **4** as a white powder in 83% yield. ³¹P NMR (CDCl₃) δ: 8.33 (br s), 62.44 (s), 62.43 (s), 62.39 (s). ¹H NMR (CDCl₃) δ: 1.28 (br s, 8H), 1.59 (t, J=7.3 Hz, 4H), 2.11 (t, J=7.6 Hz, 4H), 2.77 (t, J=7.5 Hz, 4H), 3.43 (q, J=6.9 Hz, 4H), 3.50 (d, J=13.9 Hz, 18H), 3.51 (d, J=13.7 Hz, 12H), 5.54 (t, J=7.1 Hz, 2H), 6.92 (d, J=8.2 Hz, 4H), 6.99 (d, J=8.4 Hz, 8H), 7.03 (d, J=8.4 Hz, 4H), 7.05 (d, J=6.7 Hz, 6H), 7.05 (d, J=6.4 Hz, 6H), 7.63-7.56 (m, 20H), 7.64 (d, J=2.4 Hz, 6H), 7.70 (d, J=2.1 Hz, 4H). ¹³C NMR (CDCl₃) δ: 25.59 (s), 29.05 (s), 29.14 ((s), 31.98 (d, J=12.8), 32.01 (d, J=13.0 Hz), 35.18 (s), 36.67 (s), 40.69 (s), 121.07 (s), 121.32 (s), 121.37 (s), 128.62 (s), 129.70 (s), 131.19 (s), 131.28 (s), 135.90 (s), 140.64 (d, J=18.7 Hz), 140.64 (d, J=18.7 Hz-), 140.82 (d, J=18.9 Hz), 148.82 (br s), 151.72 (br s), 151.83 (br s), 173.15 (s).

To a solution of tyraminobismethylenephosphonate HOC₆H₄CH₂CH₂N[CH₂PO₃Me₂]₂(2.81 g, 7.34 mmol) in THF (30 mL) with Cs₂CO₃ (4.77 g, 14.68 mmol) is added the dendrimer **4** (1.3 g, 0.37 mmol). After stirring overnight at room temperature, the reaction mixture is filtered through celite and then concentrated under reduced pressure. The dendrimer **5** is obtained as a colorless oil in quantitative yield. ³¹P NMR (CDCl₃) δ: 8.39 (br s), 26.73 (s), 26.74 (s), 26.76 (s), 63.12 (s), 63.17 (s). ¹H NMR (CDCl₃) δ: 1.25 (br s, 8H), 1.54 (br s, 4H), 2.07 (t, J=7.6 Hz, 4H), 2.73 (t, J=7.7 Hz, 44 H), 3.02 (t, J=7.7 Hz, 40H), 3.16 (d, J=9.5 Hz, 80H), 3.24 (d, *J*=10.1 Hz, 15H), 3.30 (d, *J*=10.2 Hz, 15H), 3.71 (d, J=10.3 Hz, 240H), 6.48 (d, *J*=5.9 Hz, 2H), 6.90 (d, *J*=8.1 Hz, 4H), 7.02- 6.95 (m, 14H), 7.05-7.02 (m, 10H), 7.12-7.06 (m, 40H), 7.20-7.12 (m, 40H), 7.62-7.57 (m, 20H), 7.63 (br s, 5H), 7.65 (br s, 5H. ¹³C NMR (CDCl₃) δ: 25.75 (s), 29.45 (s), 29.56 (s), 33.08-32.88 (m), 35.15 (s), 36.53 (s), 40.75 (s), 49.45 (dd, *J*=157.6, *J*=7.2 Hz), 53.69-51.48 (m,) 58.10 (t, *J*=7.6 Hz), 120.93 (s), 121.19 (br s), 128.27-128.21 (s), 129.60 (s), 129.91 (s), 132.05 (s), 132.14 (s), 136.57 (s), 136.57 (s), 138.88-138.65 (m), 148.93 (d, *J*=6.9 Hz), 148.90 (d, *J_{CP} =* 6.9 Hz), 151.24 (br s), 173.19 (s).

To a solution of **5** (3 g, 0.28 mmol) in anhydrous CH₃CN (150 mL) at 0 °C is added dropwise BrTMS (3.8 mL, 28.8 mmol). The reaction mixture is stirred overnight at room temperature and then the solvent is concentrated to dryness under reduced pressure. The residue is stirred for one hour in MeOH (30 mL). The resulting powder is filtered-off and successively washed twice with MeOH (20 mL) and with Et₂O (20 mL). The resulting solid is dried under reduced pressure to give the expected compound in quantitative yield. The phosphonic acid terminated dendrimer is converted to the corresponding sodium salt by adding 40 equivalent of a freshly titrated 0.1M HONa solution at 0°C. The resulting solution is filtered through a 0.4 µM microfilter and then lyophilized to give **Dendrimer 2** as a white powder in 95% yield.

³¹P NMR (D₂O/CD₃CN) δ: 7.04 (s), 9.35 (br s), 64.21 (s), 64.28 (s). ¹H NMR (D₂O/CD₃CN) δ: 2.97 (br s, 40 H), 3.20 (d, *J*=10.2 Hz, 30H), 3.29 (d, *J*=11.9 Hz, 84H), 3.62-3.44 (m, 40H), 6.86-6.79 (m, 24H), 7.05-7.01 (m, 40H), 7.26-7.21 (m, 40H), 7.56-7.40 (m, 20H), 7.73 (s, 6H), 7.76 (s, 4H). ¹³C NMR (D₂O/CD₃CN) δ: 26.49 (s), 29.75 (s), 33.49 (d, *J*=10.8 Hz), 53.87 (d, *J*=126.8 Hz), 58.54 (s), 121.55 (s), 122.04 (s,), 122.34 (d, *J*=3.7 Hz), 129.28 (s), 131.05 (s), 131.51 (s), 133.28 (s), 135.18 (s), 137.59 (s), 141.41-140.80 (m), 150.10 (d, *J*=7.2 Hz), 151.50 (br s), 176.71 (s).

### Dendrimer 3 (IMD 036)

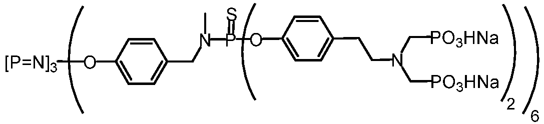

Dendrimer 3 was synthesized according to the synthetic procedure described in WO 2021/130454 A1.

### Dendrimer 4

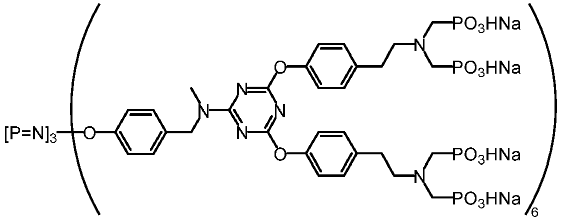

Dendrimer 4 was synthesized according to the synthetic procedure described in WO 2021/130453 A1.

### IN VIVO BIOLOCICAL EVALUATIONS

### Materials and methods

### 1. Animals

Female Sprague-Dawley rats, 7 weeks old at delivery.

### 2. Pharmacological treatment

- Dendrimer 1: 20 mg/mL- Dendrimer 2: 20 mg/mL- Dendrimer 3: 20 mg/mL
- Vehicle: Saline- Route of administration: intravesical instillation (i.ves.), 500 µL, 30 min of contact- Frequency of administration: once at D0, D2, D4 and D6 (a.m. before CYP injection)

### 3. CYP-induced chronic cystitis

The CYP rat model is fully described in C. Augé et al. Front Pharmacol. 2020, 11, 1305. CYP (prepared fresh every day) was injected intraperitoneally (i.p.) at 40 mg/kg in a final volume of 5 mL/kg in saline at D0, D3, and D6 (p.m.). Control rats received saline.

### 4. Bladder pain assessment

Bladder pain was assessed by means of mechanical stimulation using von Frey filaments as described in C. Augé et al. Front Pharmacol. 2020, 11, 1305.

### 5. Sampling

Bladders were collected and cut in 3 pieces. 2 pieces were snapped frozen and stored at - 80°C. 1 piece was transferred into tissue processing embedding cassette, fixed in 4% buffered formaldehyde and kept at room temperature for 24h. Histological samples were then transferred into ethanol 70% and stored at room temperature.

### 6. Urinary bladder inflammation assessment

### Experimental protocol

At D9, after collection, histopathological images of bladders were obtained following routine hematoxylin-eosin (H/E) staining or uroplakin staining.

### Results

Basal nociceptive responses (*i.e.* before CYP or saline injection) were similar between all experimental groups.

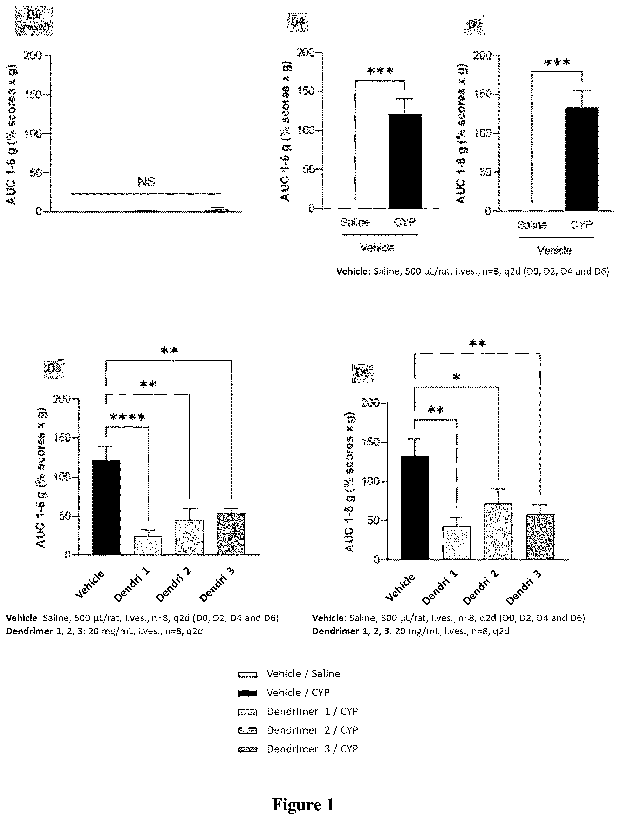

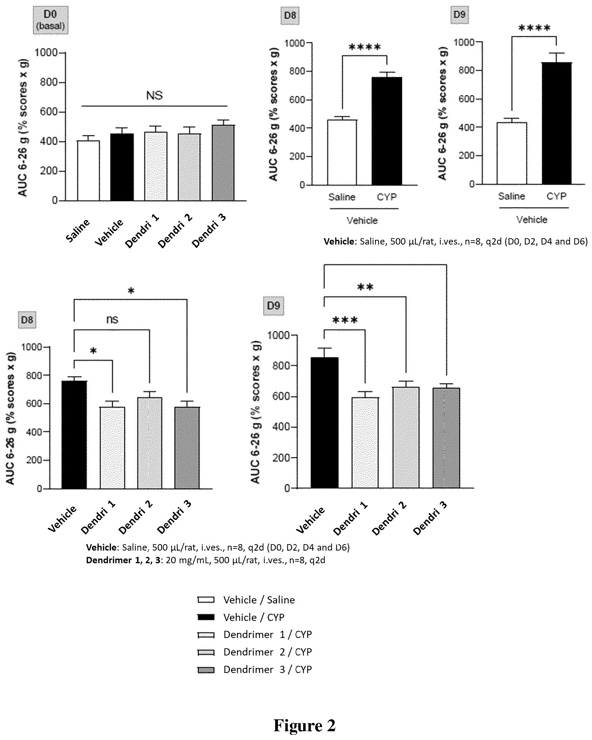

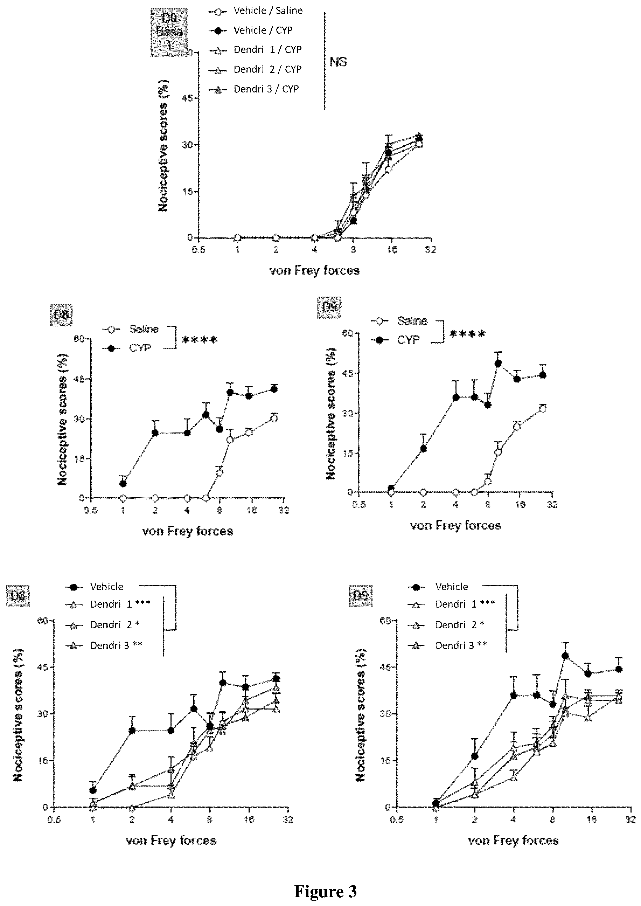

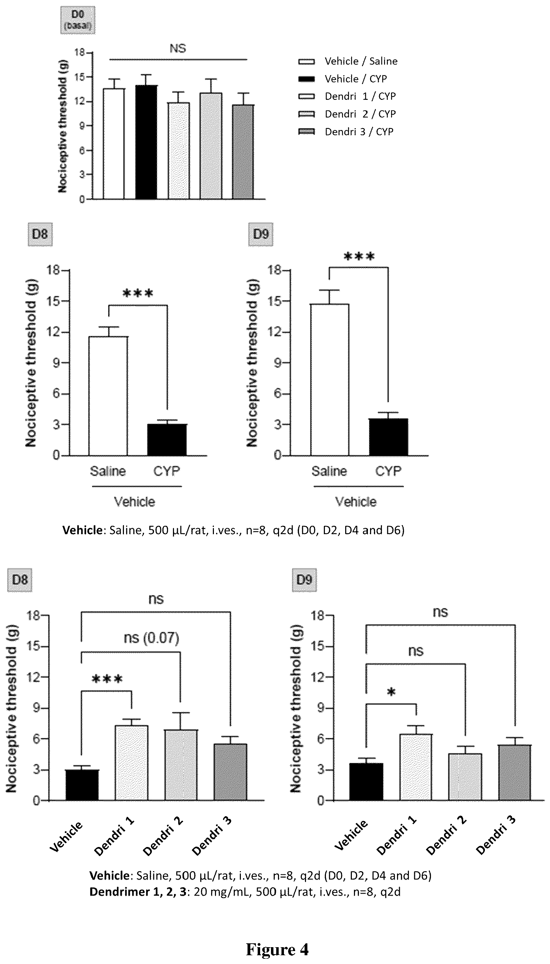

In comparison to saline, repeated injections of CYP (40 mg/kg, i.p.) induced a marked bladder pain response characterized by:
- A significant decrease in nociceptive threshold at D8 and D9 (see Figure 4),
- A significant increase in nociceptive scores at both time points (see Figure 3), and
- A significant increase in AUCs 1-6 g (allodynia) (see Figure 1) and 6-26 g (hyperalgesia) (see Figure 2) at D8 and D9.

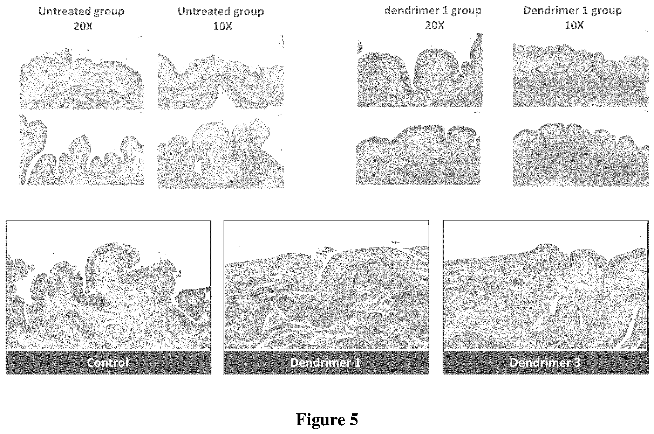

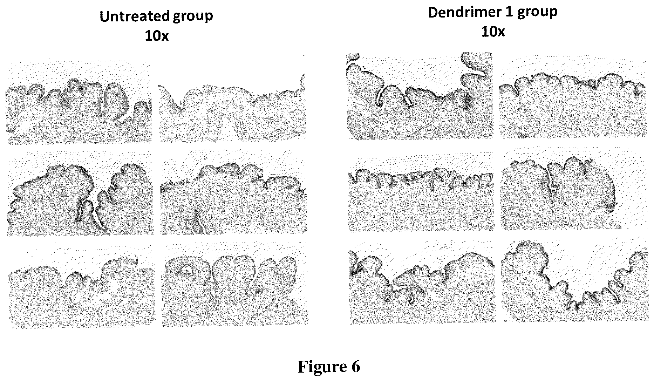

At the end of experimentation, at D9, and compared to saline, CYP induced a bladder inflammation characterized by:
- A significant increase of oedema as observed in histopathological samples (H/E staining) (see Figure 5),
- A significant degradation of the urothelial barrier traduced by a lower uroplakin staining (see Figure 6).

### Effects of the compounds of the invention

At D8 and D9, in CYP-treated rats when compared to vehicle:
Dendrimer 1 (20 mg/mL, i.ves.) led to a strong decrease in nociceptive parameters characterized by:
   - A significant increase in nociceptive threshold (see Figure 4), and
   - A significant decrease in nociceptive scores (see Figure 3) associated with a significant decrease in AUC 1-6 g (allodynia) (see Figure 1) and AUC 6-26 g (hyperalgesia) (see Figure 2).
Dendrimer 2 (20 mg/mL) elicited:
   - A tendency to increase nociceptive threshold (see Figure 4), and
   - A significant decrease in nociceptive scores (see Figure 3) associated with a decrease in AUC 1-6 g (allodynia) (see Figure 1) and AUC 6-26 g (hyperalgesia) (see Figure 2) which reached the significant level except at D8.
Dendrimer 3 (20 mg/mL) led to:
   - An increase in nociceptive threshold, and
   - A significant decrease in nociceptive scores associated with significant decrease in AUC 1-6 g (allodynia) and AUC 6-26 g (hyperalgesia).

Furthermore, Dendrimer 1 and Dendrimer 3 (20 mg/mL, i.ves.) led to a strong reduction of oedema and urothelial injury. Indeed, as shown on Figure 5, H/E staining shows that edema and urothelial injury are strongly reduced in rats treated with Dendrimer 1 and Dendrimer 3.

In addition, Dendrimer 1 led to a preservation of the urothelium integrity was observed. Indeed, as shown on Figure 6, Uroplakin staining shows that urothelial integrity is largely preserved in rats treated with Dendrimer 1.

### IN VITRO BIOLOCICAL EVALUATIONS

In vitro experiments were performed on SV-HUC-1 cell line purchased from ATCC.

### Analysis of cell number after treatment with dendrimers

Cells were plated in 8-well microscopy chamber slides (Ibidi) in either HAMF12K medium with 5% penicillin/streptomycin and 10% fetal bovine serum (Merck lot n°0001650764 - decomplemented by heating at 56°C for 30min) or in RPMI. Cells were treated for 24, 48 or 72h with Dendrimer 1 or Dendrimer 3 (0.2, 2 or 20µM) before performing an MTT test to assess cell number.

Compounds of the invention were found to have no effect on cell proliferation.

### Analysis of internalization and subcellular localization of dendrimers by confocal microscopy

Cells were plated in 8-well microscopy chamber slides (Ibidi) in either HAMF12K medium or in RPMI. 24h later, cells were stained with MitoTracker (100nM, 30min at 37°). After washing, fresh medium (HAMF12K or RPMI) was added then cells were treated with a fluorescent analogue of Dendrimer 1 or fluorescent analogue of Dendrimer 3 (20µM) and observed using a confocal microscope (63X) within the hour.

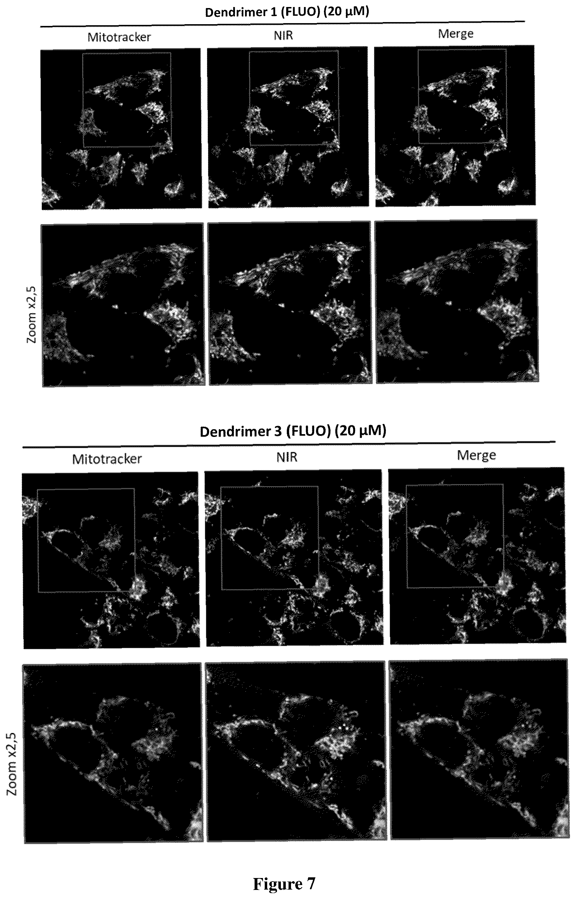

Images show that compounds of the invention were rapidly internalized by urothelial SV-HUC-1 cells and co-localize with mitochondria (see Figure 7).

## Claims

1. A compound of Formula I: 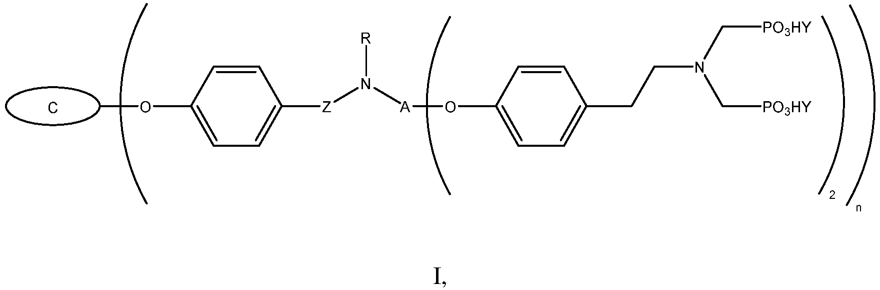 or a pharmaceutically acceptable salt thereof,
wherein 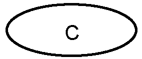 is selected from the group consisting of 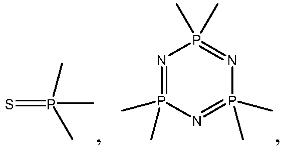 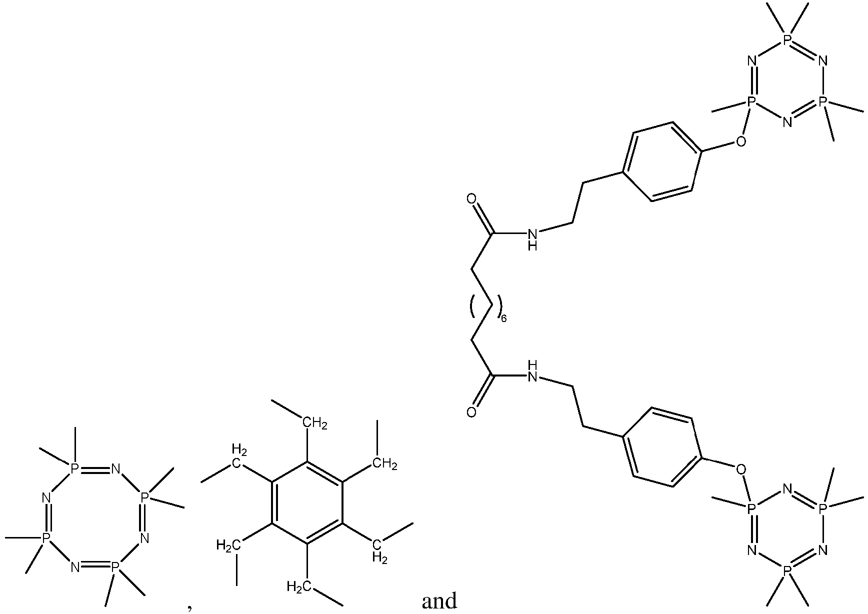
**Z** is -CH₂- or -CH=N-;
**R** is H or C1-C12-alkyl;
A is 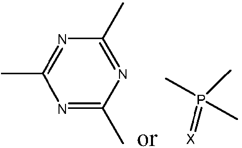 wherein **X** is S or O;
**n** is an integer from 3 to 10;
for use in the treatment and/or prevention of a disease or disorder involving bladder inflammation.

2. The compound for use according to claim 1, wherein **A** is 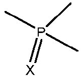 wherein **X** is S or O, preferably wherein **X** is S.

3. The compound for use according to claim 1 or 2, wherein **R** is Me.

4. The compound for use according to any one of claims 1 to 3, wherein 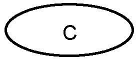 is

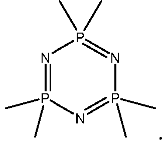

5. The compound for use according to claim 4, wherein **n** is 6.

6. The compound according to claim 1, selected from the group consisting of: 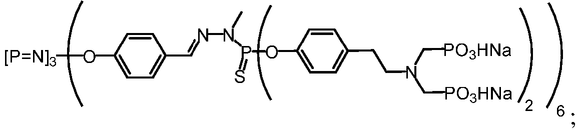 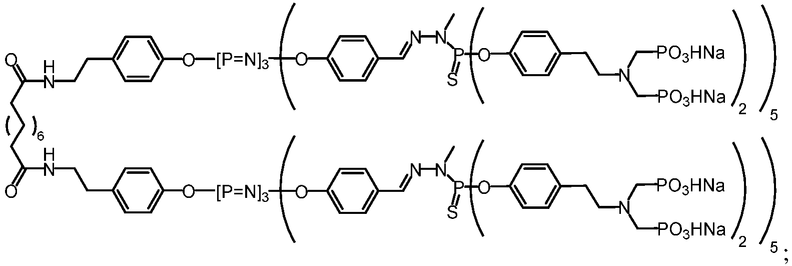 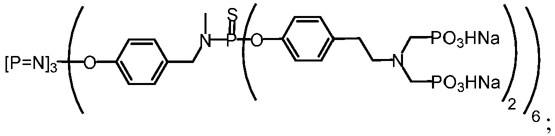 and

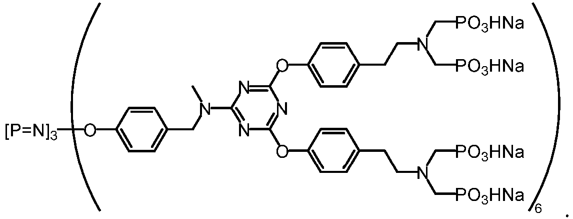

7. The compound for use according to any one of claims 1 to 6, wherein the disease or disorder involving bladder inflammation is selected from the group consisting of cystitis, urinary incontinence, overactive bladder, interstitial cystitis, bladder pain syndrome and bladder cancer.

8. A pharmaceutical composition comprising a compound of formula I as defined in one of claims 1 to 6, or a pharmaceutically acceptable salt thereof, and at least one pharmaceutically acceptable carrier, diluent, excipient and/or adjuvant, for use in the treatment and/or prevention of a disease or disorder involving bladder inflammation.

9. The pharmaceutical composition for use according to claim 8, wherein the disease or disorder involving bladder inflammation is selected from the group consisting of cystitis, urinary incontinence, overactive bladder, interstitial cystitis, bladder pain syndrome and bladder cancer.

10. A compound of Formula VI: 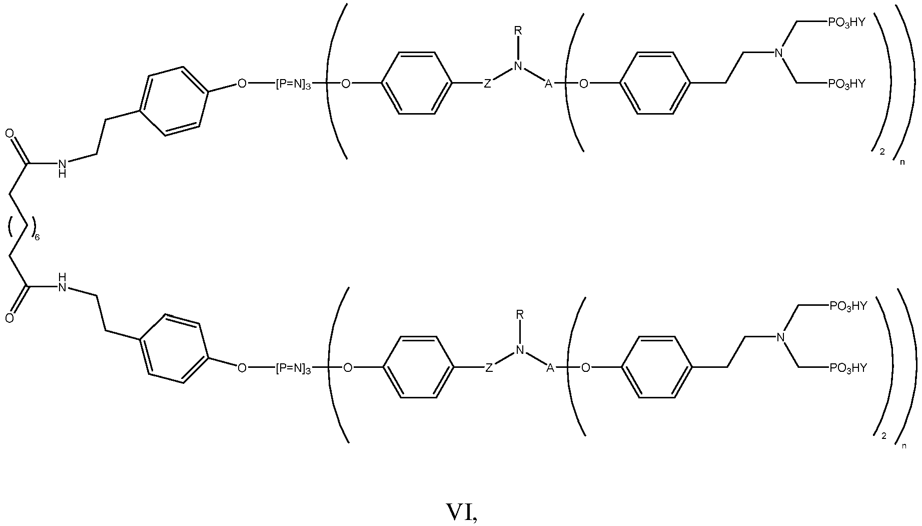 or a pharmaceutically acceptable salt thereof,
wherein
**Z** is -CH₂- or -CH=N-;
**R** is H or C1-C12-alkyl;
A is 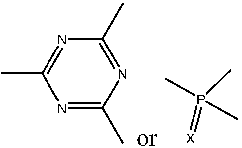 wherein **X** is S or O;
**Y** is selected from the group consisting of H, Na and K; and
**n** is an integer from 3 to 10.

11. The compound according to claim 10, wherein **Z** is-CH=N-.

12. The compound according to claim 10 or 11, wherein **A** is 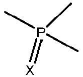 wherein **X** is S.

13. The compound according to any one of claims 10 to 12, wherein **n** is 5.

14. The compound according to claim 10, having the following formula:
